# EUROPEAN PATENT APPLICATION

(11) **EP 1 901 011 A1**
(43) Date of publication of application: **19.03.2008**
(21) Application number: 07116485.9
(22) Date of filing: 14.09.2007
(51) Int. Cl.: F24F 6/00, A61M 16/00

(54) **System and method for humidifying a breathing gas**

(30) Priority: 18.09.2006 US 522682
(71) Applicant: INVACARE CORPORATION, Elyria, OH 44036-2125 (US)
(72) Inventor: Polacsek, David, Elyria, OH 44035 (US)
(74) Representative: Ganguillet, Cyril

(57) **Abstract**

A respiratory gas treatment unit includes a humidifier with an induction water heating mechanism. A biocompatible heating target is placed in contact with humidifying liquid held in a humidifying vessel. A magnetic field is generated in close proximity to the heating target to generate heat in the target. The biocompatible heating target can be submerged within the humidifying liquid or can be formed by integrally molding ferromagnetic particles into a plastic wall or floor of the humidifying vessel.

## Description

### Background

Obstructive sleep apnea is an airway breathing disorder caused by relaxation of the muscles of the upper airway to the point where the upper airway collapses or becomes obstructed by these same muscles. It is known that obstructive sleep apnea can be treated through the application of pressurized air to the nasal passages of a patient. The application of pressurized air forms a pneumatic splint in the upper airway of the patient thereby preventing the collapse or obstruction thereof.

### Summary

A respiratory gas treatment unit includes a humidifier with an induction water heating mechanism. A biocompatible heating target is placed in thermal communication with humidifying liquid held in a humidifying vessel such that heat from the heating target acts to heat the humidifying liquid. An electro-magnetic field is generated in close proximity to the heating target to generate heat in the target. The biocompatible heating target can be submerged within the humidifying liquid or can be formed by integrally molding ferromagnetic particles into a plastic wall or floor of the humidifying vessel.

### Brief Description of the Drawings

In the accompanying drawings which are incorporated in and constitute a part of the specification, embodiments of the invention are illustrated, which, together with a general description of the invention given above, and the detailed description given below, serve to example the principles of this invention.

Figure 1 is a perspective view of a respiratory gas treatment unit constructed in accordance with an embodiment of the present invention;

Figure 2 is a perspective view of the respiratory gas treatment unit of Figure 1 with a humidifying liquid vessel removed.

Figure 3 is a rear plan view of the respiratory gas treatment unit of Figure 1.

Figure 4 is a rear view of a humidifying liquid vessel of the respiratory gas treatment unit of Figure 1.

Figure 5 is a top cross section view of the vessel of Figure 4.

Figure 6 is a side cross section view of the vessel of Figure 4.

Figures 7a-7c are perspective views of heating elements that can be used with the respiratory gas treatment unit of Figure 1.

Figure 8 is a functional block diagram of the respiratory gas treatment unit of Figure 1.

Figure 9 is a flowchart outlining one procedure for operating the respiratory gas treatment unit of Figure 1.

Figures 10 and 11 are schematic diagrams of respiratory gas treatment units constructed in accordance with alternative embodiments of the present invention.

### Detailed Description

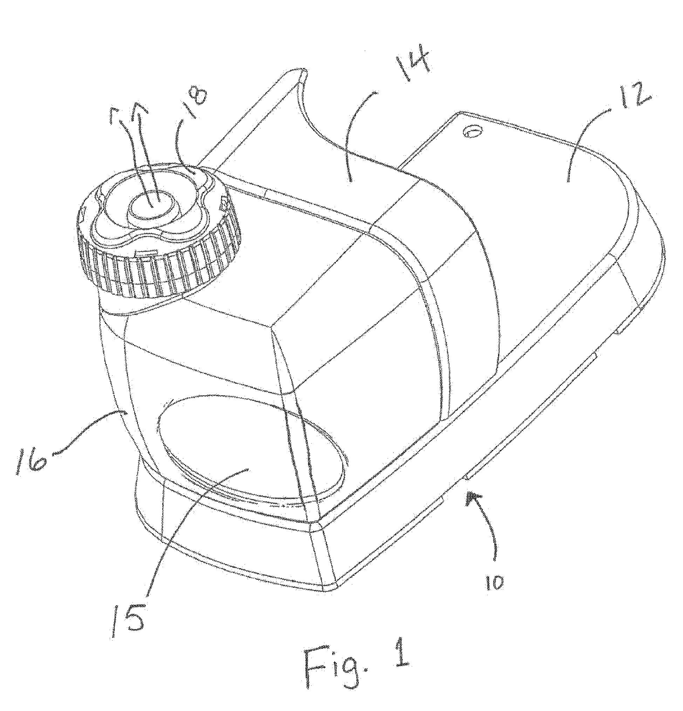

Referring to Figure 1, a continuous positive airway pressure (CPAP) respiratory treatment unit 10 is shown. For simplicity, Figure 1 shows humidifying vessel 16 attached to a base 12 and a bulkhead 14 but does not show components associated with pressurizing air such as a blower or compressor. These components are in fluid communication with the humidifier such that the pressurized air can be passed through the humidifying vessel, out of a cap 18, and through a hose worn by a patient (not shown). Liquid, such as water, present in the vessel is heated by a heating element or target 15 in which heat is induced by passing a magnetic field generated through a target within the vessel. The magnetic field is generated by a coil shaped inductor located within the base 12 under the target's position within the vessel.

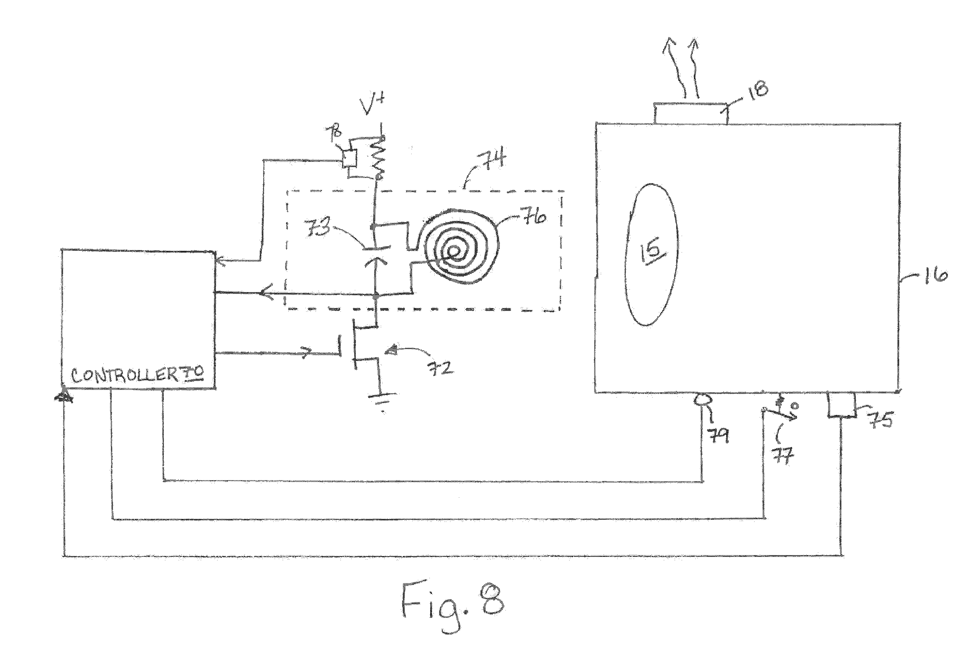

The size and shape of the target is selected based on properties of the specific magnetic field that will be generated as well as maintenance and manufacturing concerns. The target 15 shown in Figure 1 has a generally oval wafer shape, but targets of any size and shape are contemplated, such as targets 15' and 15" shown in Figures 7b and 7c which are of circular and cylindrical shape, respectively. Because it is submerged in water that will be used to humidify respiratory gas, the target is biocompatible. The target is made of a material that is selected to reduce the potential for corrosion or other reaction with the water. For example, the target may be made of stainless steel, nickel, ceramic coated ferromagnetic material, or a composite material that has favorable characteristics. The target is also sized for relatively easy removal from the vessel for cleaning. The oval shape of the target 15 allows it to be passed through the fill opening of the vessel.

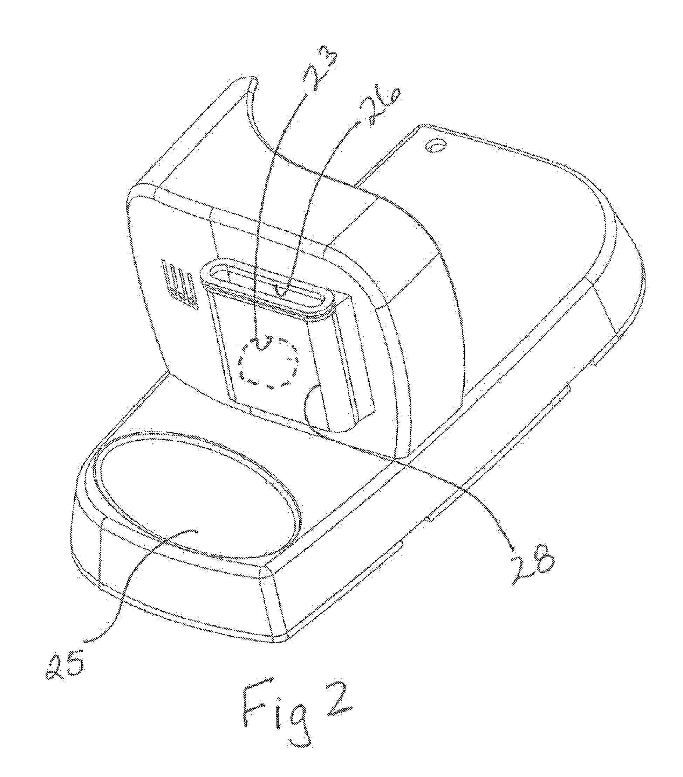

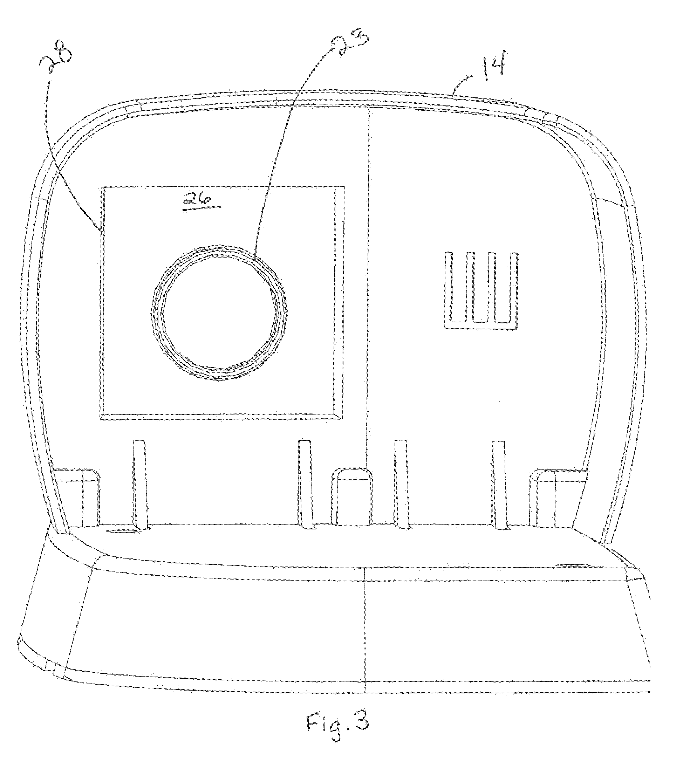

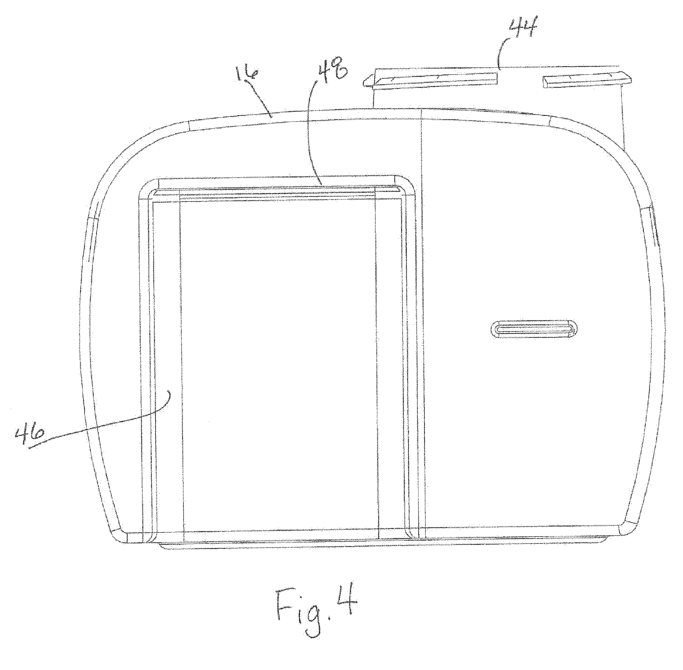

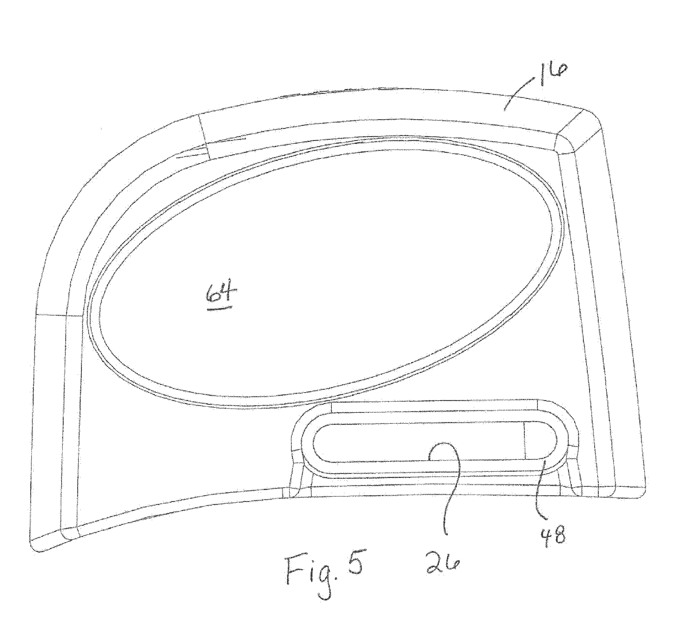

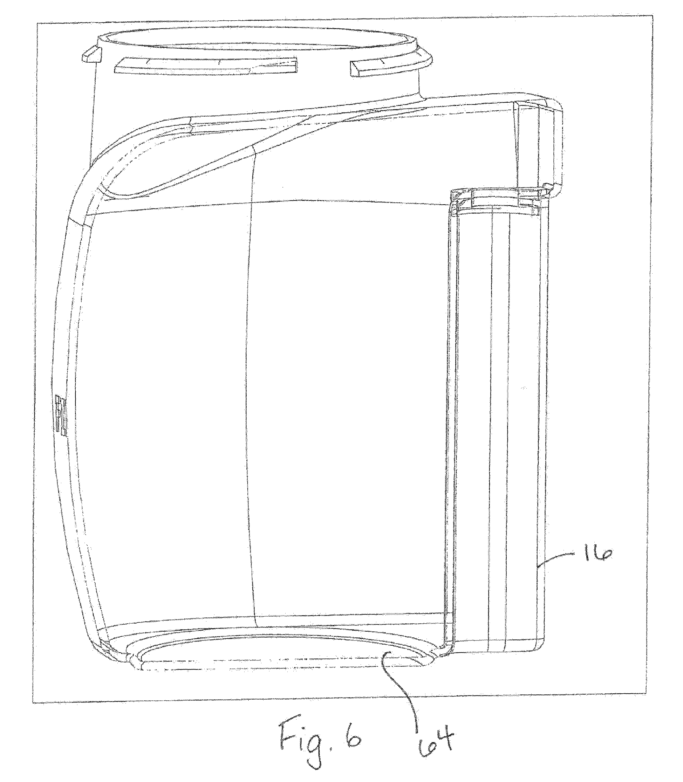

Figures 2 and 3 show the respiratory gas treatment unit with the vessel removed. A pressurized air passageway 26 is present within a mounting tab 28 that is molded as part of the bulkhead 14. Air passes from the pressurization components (not shown) through an opening 23 in the bulkhead, through the passage way 26 and into the vessel. A field generator pad 25 is disposed within the base and covers a flat inductor coil through which current is passed to create a magnetic field that will excite the target 15 and induce heat therein as will be described below in more detail.

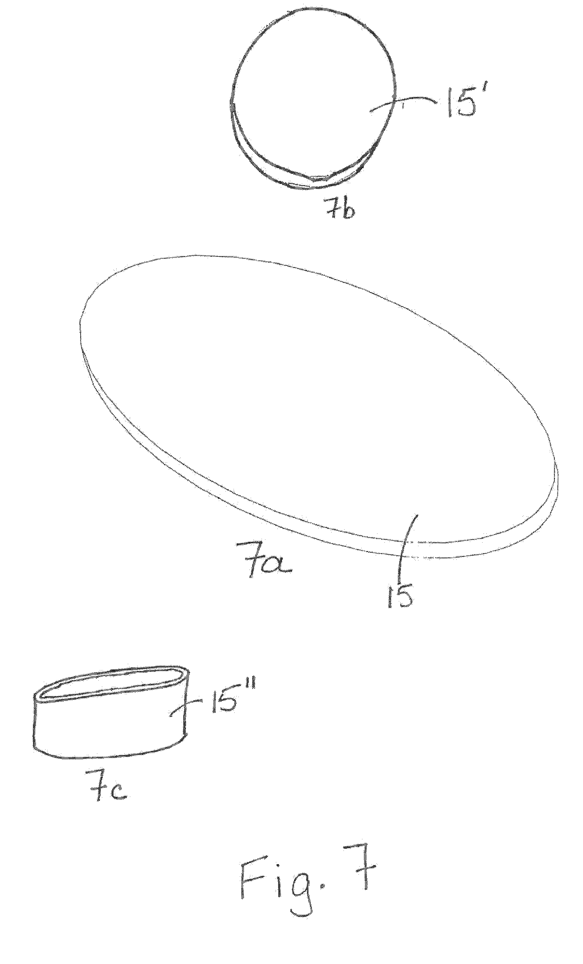

Figures 4, 5, and 6 show the vessel 16 in more detail. A fill opening 44 is present through which water or other liquid can be poured. The fill opening is adapted to accept a cap 18 (Figure 1) through which respiratory gas exits the treatment unit. A mounting channel 46 that corresponds in shape to the mounting tab 28 on the bulkhead is molded into a rear wall of the vessel. A sealing gasket 48 is pressed into the vessel at the top of the channel to seal a passageway between the bulkhead and the vessel. Referring to Figure 5, the gasket 48 can be seen in more detail. A target receiving depression 64 is molded into the floor of the vessel that is aligned with the field generator pad. The target rests in this depression when installed and is thereby placed in a predetermined location which is in close proximity to the field generating coil. The target locating depression 64 can also be seen in cross section view Figure 6.

Figure 8 is a schematic diagram of the humidifying vessel, target, and field generator and electronics. The magnetic field that is used to excite the target is generated by a resonant circuit 74 made up of a capacitor 73 and an inductor 76. A controller 70 selectively powers the resonant circuit by activating a switching element 72, such as MOSFET, transistor, or other device, to allow current to flow through the resonant circuit. The switching element is switched at the resonant frequency of the resonant circuit and is connected to allow the voltage source to replenish energy lost due to the magnetic coupling with the target. A current monitor 78 monitors the current passing through the resonant circuit and current data from the monitor is used by the controller 70 to switch the switching element 72 at the appropriate frequency. The resulting induction heating of the humidification liquid utilizes electrical energy that is converted into rapidly oscillating electromagnetic waves by the resonant circuit 74 to generate a magnetic field and this magnetic field is then applied to a ferromagnetic material in the target 15. This ferromagnetic material then heats up due to energy dissipation in the material due to the interaction of the magnetic field and the hysteresis of the ferromagnetic material. Hysteresis is the resistance of a material to changes in a magnetic field in the material. The magnetic field produced in a coil also induces eddy currents in the magnetic material. This eddy current in the magnetic material is converted into heat due to the resistance of the material to the magnetic field changes and the eddy current flow in the material. The heat generated in a ferromagnetic material is then coupled directly to water being heated for humidification purposes by placing the ferromagnetic material in the water.

The electrical control circuitry consists of elements that take electrical energy and generate a rapidly oscillating electrical current. This oscillating electrical current is flows through a resonant circuit 74 consisting of an inductor 76, which is the coupling coil, and the capacitor 73. This resonant circuit then sets up a rapidly oscillating magnetic field external to the inductor coils due to electromagnetic interaction of the current in the inductor. This external magnetic field then induces current flow and resultant heat in an external conducting material brought into close proximity to the magnetic field.

The ferromagnetic heating target 15 is a material or combination of materials that convert a rapidly oscillating magnetic field into heat. The material or materials will heat up due to hysteresis losses caused by resistance of the material to changes in the magnetic moments of the atoms making up the structure. Additional heating can be generated in the material due to eddy currents that are generated in the inherent resistance of the material. The rapidly oscillating magnetic field will induce circulating electrical currents in the material structure and these will be dissipated as heat.

In normal operation, the vessel 16 is filled with water, the ferromagnetic heating target 15 is placed in the water chamber, and the water chamber is then placed in close proximity to the resonant coil assembly. Energizing the resonant coil assembly 74 with an oscillating electrical field from the control circuitry 72 then induces an oscillating magnetic field in the ferromagnetic heating element. The resonant frequency of the circuit should be in the 20-40 kHz. range or higher to maximize the energy transfer as far into the ferromagnetic heater element as possible.

The heating of the ferromagnetic heating target due to eddy currents and hysteresis heating then transfers the heat energy directly to the water molecules. This raises the water temperature, raising the relative humidity of the treatment air stream flowing over the water surface. There is no direct contact of any surface of the humidification chamber with any high temperature source of external heat that is typically employed in most indirectly heated humidifiers. The heat energy is transferred directly from the resonant coil assembly to the ferromagnetic heating element and then to the water. The heating efficiency of the energy conversion from electrical power to water temperature is improved by directly heating the water molecules without intermediate heat losses. There are minimal electrical conversion losses associated with the induction heating system.

The induction heating system can be applied to any humidifier or system used to add relative humidity to a treatment air stream. This can include and not limited to hospital ventilation systems, home care ventilators, sleep apnea therapy systems, or portable ventilation systems used in transport or emergency care.

Figure 8 also shows a thermal sensing probe 79 that monitors an exterior temperature of the vessel to provide an indication of water temperature. The temperature of the water can be monitored and controlled by several means. One means is using a temperature probe in direct contact with the water used for humidification. This technique does need a probe located in the water and therefore should be electrically and biomedically isolated from the water supply and possible patient contact. It also should have electrical connections to the control or measuring circuitry and therefore should be part of the humidifier assembly. Another means is to control the amount of power delivered to the electrical resonant circuit and therefore controlling the amount of heat delivered to the water. This would be an open loop control system with no feedback. A third means is to use a non-contact optical probe to measure the temperature of the water or the ferromagnetic heater element. A non-contact probe can be a laser temperature reading circuit that is adjusted to monitor the heater element temperature. Another non-contact probe is a thermopile. A heat sensing thermopile uses the long-wavelength energy emitted by a body at a temperature. Another non-contact mechanism is to use a temperature sensitive liquid crystal assembly in the water and measure the temperature indicator position for temperature feedback. Any one of these techniques could be used in a closed loop control system. A fourth means would be to place a thermal probe in direct contact with an outside wall or floor of the humidification chamber. Sensing temperature of the water container wall would give a direct indication of the temperature of the water in the container. The temperature sensor could be a thermistor, a thermocouple, an RTD device, a semiconductor temperature sensor, or any other temperature sensing technology. This could be part of a closed loop control system for maintaining temperature regulation in the water.

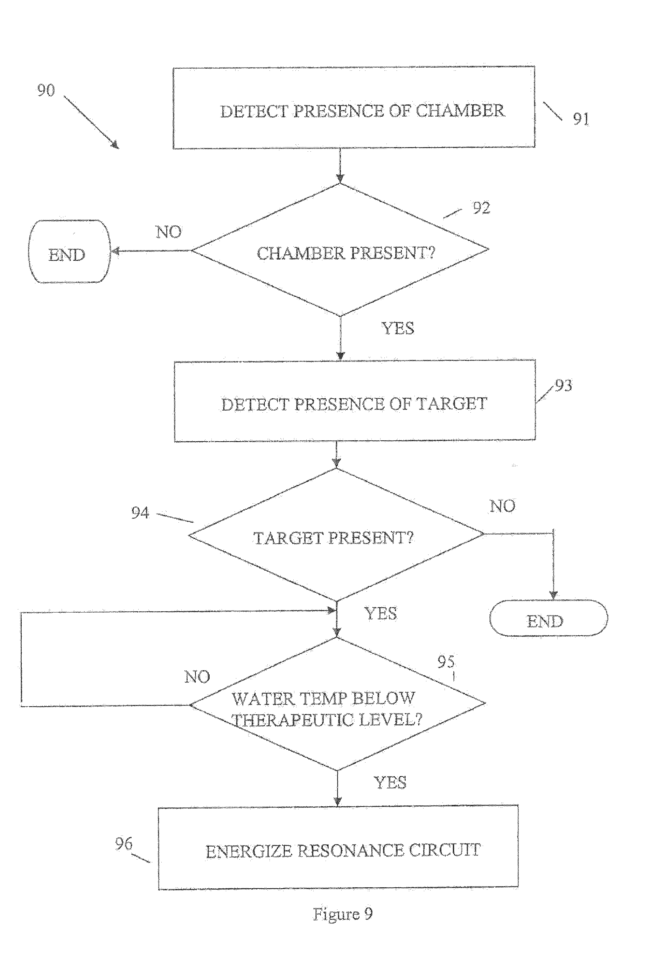

The overall operation of the resonant coil assembly can be protected against circuit failure by adding an overtemperature switch or fuse 77 to the coil assembly. An additional feature that is used to control operation of the treatment unit is a vessel sensing component (shown schematically as 75). The switch or sensor provides a signal to the controller 70 to indicate that the vessel is properly installed in the treatment unit. This signal may be used to enable operation of the treatment unit as will be outlined in connection with Figure 9.

Figure 9 is a flowchart that outlines a procedure 90 that can be used to operate the field generator when the treatment unit has been switched on by a patient. At 91, the presence of the chamber is detected and at 92 if the chamber is not present, the procedure ends and the resonance circuit is not energized. If the chamber is present at 93, the controller briefly energizes the resonance circuit to "ping" for the presence of the target. The target is detected by monitoring changes in the behavior of the resonance circuit, such as an increase in current passing through the current monitor 78 (Figure 8), that result from the presence of the target within the magnetic circuit through which the field passes. If the target is present, but out of position, it will not create the correct change in circuit behavior and in either case at 94 the procedure ends. If the target is present and in position, at 95 the procedure checks the water temperature. If the temperature is below a predetermined therapeutic level at 96 the resonance circuit is energized until the water has heated to the predetermined therapeutic temperature.

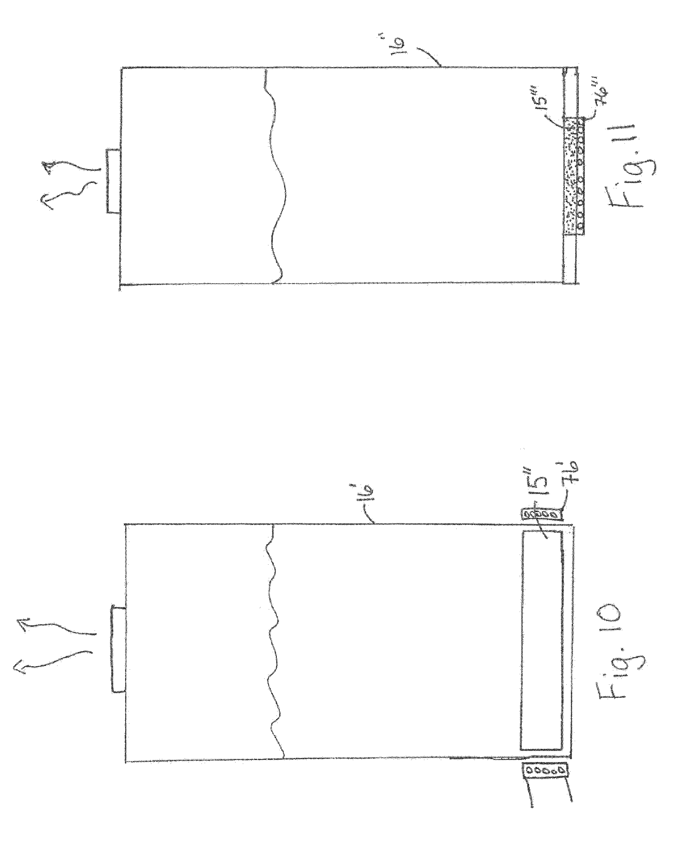

Figure 10 is a schematic diagram of vessel 16', field generator 76', and target 15" that is also shown in Figure 7. In this construction, the vessel has a generally cylindrical shape and the target 15" is of corresponding cylindrical shape. The target can be removable from the vessel as shown, or it may be integrally molded within the walls of the vessel. The field generator 76' includes a coil into which the vessel is inserted, placing the target within the coil to promote efficient induction of heat. Figure 11 shows yet another arrangement in which the vessel 16" has a floor into which ferromagnetic particles are integrally molded to form a target 15"'. This arrangement requires less patient maintenance because the target is molded within the vessel and can be cleaned at the same time as the vessel.

While the present invention has been illustrated by the description of embodiments thereof, and while the embodiments have been described in considerable detail, it is not the intention of this specification to restrict or in any way limit the scope of the appended claims to such detail. Therefore, the invention, in its broader aspects, is not limited to the specific details, the representative apparatus, and illustrative examples shown and described. Accordingly, departures may be made from such details without departing from the spirit or scope of the applicant's general inventive concept.

## Claims

1. A humidifier for pressurized respiratory therapy gases comprising:
a vessel that holds a humidifying liquid and includes a respiratory gas outlet through which pressurized respiratory gases can exit;
a biocompatible ferromagnetic heating element in thermal communication with the humidifying liquid; and
a magnetic field generator that is magnetically coupled to the ferromagnetic heating element.

2. The humidifier of claim 1 wherein the biocompatible ferromagnetic heating element has a generally oblong shape.

3. The humidifier of claim 1 or 2, wherein the magnetic field generator comprises:
an electrical control circuit that generates an oscillating current; and
a resonant circuit that is powered by the oscillating current to produce an oscillating magnetic field.

4. The humidifier of claim 3 wherein the resonant circuit comprises a coil through which the oscillating current is passed.

5. The humidifier of claim 4 wherein the resonant circuit comprises a thermal protection device.

6. The humidifier of claim 5 wherein the thermal protection device is a fuse.

7. The humidifier of claim 5 wherein the thermal protection device is a thermally activated switch.

8. The humidifier of any one of claims 1-7 comprising a liquid temperature monitor.

9. The humidifier of claim 8 wherein the liquid temperature monitor is a temperature probe that is submerged in the humidifying liquid.

10. The humidifier of claim 8 wherein the liquid temperature monitor is laser temperature reading circuit.

11. The humidifier of claim 8 wherein the liquid temperature monitor is a thermopile.

12. The humidifier of claim 8 wherein the liquid temperature monitor is a temperature probe that is in contact with an outside surface of the vessel.

13. The humidifier of any one of claims 8-12 comprising a controller that controls the magnetic field generator based on signals from the fluid temperature monitor.

14. The humidifier of any one of claims 1-13 wherein the biocompatible ferromagnetic heating element is made of a ceramic coated ferromagnetic material.

15. The humidifier of any one of claims 1-13 wherein the biocompatible ferromagnetic heating element is made of stainless steel.

16. The humidifier of any one of claims 1-13 wherein the biocompatible ferromagnetic heating element is made of nickel.

17. The humidifier of any one of claims 1 - 16 wherein the vessel includes one or more heating element locating features that maintain the heating element in proper operating orientation.

18. The humidifier of claim 17 wherein the heating element locating features include an indentation of corresponding shape to the heating element molded into a floor of the reservoir.

19. The humidifier of any one of claims 1-18 wherein the biocompatible ferromagnetic heating element is cylindrically shaped and fits closely within vessel walls and wherein the magnetic field generator includes a coil that closely surrounds the vessel outside a location of the cylindrically shaped heating element.

20. The humidifier of any one of claims 1-18 wherein the vessel is molded of plastic and is defined by one or more vessel walls and a vessel floor and wherein at least a portion of one of the vessel walls or floor includes a heating element formed of integrally molded ferromagnetic particles within the plastic material.

21. The humidifier of any one of claims 1 - 18 wherein the ferromagnetic heating element is removable from the vessel.

22. The humidifier of any one of claims 3 - 21 wherein the resonant circuit includes a thermal protection device in series with the oscillating current.

23. The humidifier of any one of claims 1 - 18 wherein the biocompatible ferromagnetic heating element has a generally circular wafer shape.

24. A system for providing pressurized, humidified gas to a patient, comprising:
the humidifier according to any one of claims 1 to 23; and,
a blower.

25. A method for humidifying respiratory gas with a humidifier comprising:
disposing a biocompatible ferromagnetic heating element in thermal communication with humidifying liquid in a humidifying vessel;
transmitting a magnetic field to the ferromagnetic heating element to induce heating in the ferromagnetic heating element to heat the humidifying liquid to a therapeutic temperature; and
passing the respiratory gas through the vessel.

26. The method of claim 25 wherein the step of transmitting a magnetic field through ferromagnetic heating element is performed by powering a resonant circuit with an oscillating current.

27. The method of claim 25 or 26 comprising verifying a proper installation of the vessel within the humidifier prior to transmitting the magnetic field.

28. The method of any one of claims 25 - 27 comprising verifying a proper location of the heating element by briefly transmitting the magnetic field and monitoring a change in current through the resonant circuit in response to transmission of the magnetic field.

29. The method of any one of claims 25 - 28 comprising monitoring a demand for respiratory gas and wherein the step of transmitting a magnetic field is performed for a limited duration when no demand for respiratory gas is detected.
